# FASCICULE DE BREVET EUROPEEN

(11) **EP 2 632 404 B1**
(45) Date de publication et mention de la délivrance du brevet: **01.09.2021**
(21) Numéro de dépôt: 11799473.1
(22) Date de dépôt: 26.10.2011
(51) Int. Cl.: A61F 7/10, A61F 7/02, B65B 9/04, A61F 7/00

(54) **PROCEDE ET INSTALLATION DE FABRICATION DE COMPRESSES A EFFET REFROIDISSANT ET COMPRESSES OBTENUES EN CONDITIONNEMENT STERILE**
VERFAHREN UND ANLAGE ZUR HERSTELLUNG VON KOMPRESSEN MIT KUEHLEFFEKT UND KOMPRESSEN IN STERILER VERPACKUNG
METHOD AND INSTALLATION FOR PRODUCTION OF COMPRESSES HAVING A COOLING EFFECT, AND COMPRESSES OBTAINED IN STERILE PACKAGING

(30) Priorité: 26.10.2010 FR 1004207; 26.10.2010 FR 1004206
(43) Date de publication de la demande: 04.09.2013
(73) Titulaire: Boiron, 69510 Messimy (FR)
(72) Inventeur: CACERES, Patrick, F-69110 Ste Foy les Lyon (FR); CACERES, Franck, F-69110 Ste Foy les Lyon (FR)
(74) Mandataire: Plasseraud IP
(86) Numéro de dépôt international: PCT/IB2011/002567
(87) Numéro de publication internationale: WO 2012/056308

(56) Documents cités:
- EP-A1- 1 607 074
- WO-A2-2007/035312
- US-A- 3 587 578
- US-A- 5 313 809
- US-A1- 2004 149 732
- US-B1- 6 823 649

## Description

La présente invention concerne la fabrication industrielle d'articles de soin à base de compresses à effet refroidissant par gonflement à l'eau.

Dans le domaine des articles à usage médical notamment, on connaît de telles compresses, dont le fonctionnement est dû à l'activité cryogénique dont sont capables des particules de polymères absorbants ayant retenu de grandes quantités d'eau. On parle de polymères actifs en réfrigération par absorption d'eau, ou de polymères activés par gonflement à l'eau, sachant que l'effet cryogénique se produit essentiellement par vaporisation de l'eau lors de sa désorption hors des particules absorbantes.

On connaît de l'art antérieur, et notamment de la demande de brevet européen EP 1 607 074, de telles compresses à base de polymère absorbant, activable à l'eau, qui se présentent à l'état sec incluses en conditionnement stérile avec une réserve d'eau d'activation en sachet, dans un article de soin, notamment à usage de pansement médical, en préservant l'état des éléments qui le composent dans une enveloppe stérile jusqu'au moment où l'on provoque la rupture du sachet d'eau et le gonflement du polymère dans la compresse proprement dite en vue de son utilisation.

La présente invention décrit dans les revendications indépendantes 1,3 et 12 un procédé de fabrication d'article à compresses à effet refroidissant, une installation de fabrication de ces articles et l'article lui-même respectivement. Des modes de réalisation préférés du procédé, de l'installation et de l'article sont décrits dans les revendications dépendantes. La présente invention vise à permettre la fabrication en série d'articles ainsi constitués, dans des conditions compatibles avec une production à l'échelle industrielle, notamment en termes de rapidité, de coût et de fiabilité. Elle se propose aussi par là d'apporter des perfectionnement à la réalisation des articles eux-mêmes en améliorant leurs propriétés, notamment pour ce qui concerne la qualité des compresses dans leur effet réfrigérant et la sécurité d'emploi.

A titre principal, les caractéristiques propres à la présente invention concerne la fabrication des compresses elles-mêmes, sous la forme de polymère absorbant encore à l'état sec retenu dans une paroi perméables à l'eau. D'autres caractéristiques viennent avantageusement la compléter concernant leur assemblage chacune avec un sachet d'eau fracturable dans une enveloppe de conditionnement sous vide. On retiendra en outre que parmi les caractéristiques propres à la présente invention, il en est qui ont intérêt pour le procédé et l'installation de fabrication des articles, mais aussi pour les propriétés des articles considérés pour eux-mêmes. Tel est le cas notamment des particularités touchant à la nature des feuilles enfermant le polymère dans la compresse ou à celle des parois de l'enveloppe de conditionnement stérile de l'article fini avec la disposition relative entre sachet d'eau et compresse dans cette enveloppe.

Que ce soit en termes de procédé ou en termes d'installation, l'invention prévoit essentiellement de procéder par laminage entre deux feuilles en une matière de rétention des particules de polymère absorbant mais perméable à l'eau pour fabriquer en série des compresses dites sèches contenant le polymère à l'état sec réparti dans plusieurs compartiments pour former ensemble une compresse plate.

Pour l'une des feuilles, celle de préférence qui est utilisée en feuille inférieure en cours de fabrication des compresses, on choisit avantageusement une matière à déformation thermique élastiquement réversible. Alors que pour l'autre feuille, de préférence celle passant en feuille supérieure lors des opérations de fabrication, on a intérêt à privilégier la résistance mécanique, en particulier en résistance à l'allongement sous traction pendant le laminage, quitte à perdre sur la perméabilité de la paroi correspondante dans la compresse sèche obtenue. C'est alors la face de la compresse occupée par la feuille élastiquement déformable à chaud et plus perméable que l'on choisira pour se placer au mieux contre le sachet d'eau dans l'article fini.

Sous ses formes de mise en œuvre préférées, l'invention prévoit aussi de faire appel aux techniques de laminage dans une unité de conditionnement réalisant la fabrication en série des articles finis pour commercialisation, où chaque compresse sèche est enfermée avec son sachet d'eau dans une enveloppe sous vide. Ladite enveloppe est alors constituée à partir de deux feuilles en défilement continu qui sont en une matière étanche à l'eau comme à l'air qui sont soudées ensemble autour de chaque ensemble sachet d'eau et compresse sèche l'un sur l'autre. Dans les modes de mise en œuvre préférés de l'invention, la feuille défilant en feuille inférieure dans le processus de fabrication est à déformation plastique à chaud, de sorte que l'on y ménage des cuvettes de réception des sachets d'eau supportant chacun la compresse associée. Alors que pour la feuille servant de feuille supérieure de laminage on a intérêt à privilégier les propriétés mécaniques de résistance à toute déformation. Non seulement on favorise ainsi de bonnes conditions de mise en œuvre du procédé de fabrication dans l'unité de conditionnement, mais en outre on obtient un résultat avantageux pour les conditions d'utilisation de l'article fini. En effet, il sera facile à l'utilisateur l'assurer la rupture du sachet et la libération de l'eau en agissant sur la paroi de l'enveloppe relativement rigide provenant de cette feuille supérieur, en particulier quand elle se trouve alors mitoyenne avec le sachet d'eau.

On s'intéressera maintenant à préciser la constitution d'une installation suivant l'invention ainsi que le procédé correspondant en se référant à des modes de réalisation préférés de l'invention, qui cependant ne sont pas limitatifs.

L'installation comporte principalement une unité de fabrication de compresses sèches adaptées à absorber de l'eau pour produire l'effet refroidissant, dans laquelle on fait passer sous une trémie d'alimentation en poudre de particules absorbantes une feuille inférieure de matière perméable à l'eau (avantageusement une matière textile) qui est déroulée de manière continue le long de l'unité et qui est entraînée à plat par laminage vers la sortie de l'unité et on dépose des doses successives de ladite poudre dans des sillons formés longitudinalement dans ladite feuille inférieure par déformation élastiquement réversible de celle-ci. Puis on recouvre l'ensemble d'une feuille supérieure (avantageusement également en matière textile et perméable à l'eau) qui est déroulée de manière continue et qui est entraînée à plat par laminage simultanément avec la bande de feuille inférieure vers la sortie de l'unité.

Ainsi on assure au cours du défilement la bonne répartition du polymère sur toute la surface de chaque compresse, d'une part en direction transversale au défilement par la présence de différents sillons côte à côte, d'autre part dans la direction longitudinale du défilement par le fait que chaque dose de poudre délivrée se trouve automatiquement étalée dans ce sens, chacune sur une étendue correspondant à une compresse.

Selon une autre caractéristique de l'invention, on isole transversalement les uns des autres les sillons adjacents remplis de poudre en soudant ensemble la feuille supérieure et la feuille inférieure le long des intervalles entre sillons adjacents et on ferme dans chaque sillon des compartiments successifs contenant chacun une dose de poudre en soudant l'une avec l'autre les feuilles suivant des lignes de soudure transversales en travers de l'ensemble en défilement.

Ainsi, tout de suite après le dépôt de poudre, on délimite des compartiments pour s'assurer que la poudre sera correctement répartie uniformément sur toute l'étendue de chaque compresse. On notera ici, que dans la compresse finie, les sillons sont estompés, du fait qu'en fin de l'unité de fabrication de compresses sèches, la feuille inférieure tend à reprendre élastiquement sa forme plane d'origine.

Les deux feuilles laminées constituent après soudage l'enveloppe de rétention des particules absorbantes. Elles sont donc réalisées perméables à l'eau. On utilise avantageusement un matériau textile non tissé. En pratique on s'attache à ce que l'une des deux feuilles soit de matière perméable à l'eau, alors que l'autre peut être en non tissé moins lâche, et donc plus résistante. Dans ce cas, on utilise de préférence la feuille plus perméable à l'eau comme feuille inférieure, car sa constitution lui confère alors une souplesse qui est favorable pour la mise en forme du sillon de réception de la poudre.

Selon une caractéristique de l'invention, les sillons dans lesquels la poudre va être déposée sont formés élastiquement dans la feuille inférieure par des doigts distincts les uns des autres disposés en ligne en travers du défilement de la feuille inférieure de la compresse, lesdits doigts chauffant la feuille inférieure tendue et la déformant mécaniquement, de façon élastique. On réalise ainsi une déformation temporaire de la feuille jusqu'à ce qu'après avoir reçu la poudre elle tendre à revenir à sa forme d'origine. On prévoit un doigt pour chaque sillon souhaité,

Selon une caractéristique de l'invention, l'étape de dépôt de poudre est réalisée par un ensemble de doseurs espacés l'un par rapport à l'autre pour que chaque doseur soit situé au-dessus de l'un des sillons de la feuille inférieure en défilement. Cette position des doseurs permet un dépôt de poudre maîtrisable et répétable, pour s'assurer que la même quantité de poudre sera présente dans chaque compresse et dans chaque sillon.

Selon une caractéristique de l'invention, on prévoit lors des opérations de soudure des moyens de refroidissement à buses de soufflage d'air et on s'assure que les moyens de refroidissement soient munis d'un déflecteur pour protéger les sillons remplis de poudre des jets d'air soufflé, et pour conserver ainsi la bonne répartition de la poudre.

En outre, l'installation selon l'invention comporte une unité de conditionnement faisant suite à l'unité de fabrication de compresse sèche.

Dans cette unité, on réalise par formage thermique des barquettes individuelles de réception de compresses sèches dans une première feuille en matière étanche à l'eau et à l'air et qui est entraînée à plat vers la sortie de l'unité pour y déposer l'un par dessus l'autre un sachet d'eau réalisé par ailleurs et une compresse sèche fabriquée précédemment.

On recouvre l'ensemble par une deuxième feuille en matière étanche à l'eau et à l'air qui est entraînée à plat vers la sortie de l'unité simultanément à la feuille inférieure et on ferme les bords autour de chaque barquette par soudure des feuilles l'une sur l'autre en faisant préalablement le vide entre les feuilles.

Le fait que les deux feuilles .formant parois de l'enveloppe de conditionnement soient étanches à l'eau permet d'assurer que l'eau qui va sortir du sachet va être utilisée entièrement pour imbiber la compresse. Le fait que ces feuilles soient étanches à l'air et que le vide soit fait entre les feuilles avant le collage assurent un environnement isolé de l'humidité de l'air jusqu'à ce que l'enveloppe soit ouverte pour récupérer la compresse imbibée. Dans le cas d'une fabrication réalisée dans des conditions stériles, l'étanchéité des feuilles et la mise sous vide permettent d'assurer la conservation des conditions stériles jusqu'à l'ouverture de l'enveloppe.

Selon une caractéristique de l'invention, la réalisation par formage thermique des barquettes est dimensionnée pour qu'une compresse sèche et un sachet d'eau associé soient entièrement contenus dans le volume de ladite barquette.

On s'assure alors que la compresse et le sachet ne bougent plus dès lors qu'ils sont mis en place dans la barquette, et on protège le sachet d'eau à l'intérieur de cette barquette pour éviter qu'il ne rompe sans intention volontaire de l'utilisateur.

On peut prévoir des moyens de maintien en position du sachet d'eau pour s'assurer de garder une position centrale du sachet par rapport à la compresse dans l'enveloppe de conditionnement sous vide.

Les sachets d'eau peuvent également être réalisés dans l'installation, dans une unité avantageusement parallèle à l'unité de fabrication de compresses sèches.

Dans cette unité de fabrication de sachets d'eau, qui est disposée en amont de l'unité de conditionnement, on amène et l'on soude l'un contre l'autre les deux bords latéraux d'une feuille en matière plastique pour former un tube fermé sur lui-même par soudure le long d'une génératrice, cette soudure latérale des deux bords étant réalisée pour d'une part assurer la fermeture étanche du sachet pendant tout le temps des opérations de fabrication et résister aux manipulations des sachets et d'autre part pour céder, dans le produit fini, dès que l'on appuie à plat sur l'article et donc sur le sachet d'eau, afin que l'eau vienne imbiber la compresse. On définit ainsi une zone frangible adaptée à céder par la suite quand l'utilisateur souhaite imbiber d'eau la compresse sèche pour activer son effet refroidissant sur une plaie.

Une fois cette soudure latérale effectuée, on place le tube ainsi formé dans des mâchoires de soudure thermique en transversal de l'axe du tube et on réalise une soudure transversale de ce tube qui va former la ligne de soudure inférieure du sachet. On fait ensuite s'écouler de l'eau sous pression par doses successives au centre du tube de sorte que le tube se remplisse d'eau. On vérifie le niveau de remplissage d'eau dans le tube par une sonde sensible au niveau de l'eau, puis on réalise la soudure transversale supérieure en formant alors un sachet qui enferme l'eau. On fait attention à ce que la ligne de soudure supérieure soit faite suffisamment en dessous du niveau supérieur de l'eau dans la poche formée entre les feuilles. Ainsi, lors de la soudure, on chasse l'eau en excédent et on s'assure que l'on n'enferme pas des bulles d'air dans le sachet, et d'autre part que la quantité d'eau dans le sachet est bien la quantité requise, nécessaire à la bonne imprégnation de la compresse. Dans une production de sachets d'eau en continu, le sachet ainsi formé est découpé et dégagé de l'installation pour être par la suite disposé dans l'enveloppe de conditionnement. La ligne de soudure supérieure du sachet précédent forme alors la ligne de soudure inférieure du sachet suivant et le processus de remplissage, de soudure et de découpe se poursuit cycliquement.

Selon une caractéristique de l'invention, on prévoit le transfert de chaque compresse sèche fabriquée par l'unité de fabrication de compresses sèches et le transfert de chaque sachet d'eau en sortie de l'unité de fabrication de sachets pour transférer un sachet et une compresse dans une barquette préformée dans l'enveloppe de conditionnement au niveau de l'unité de conditionnement.

On prévoit en outre dans cette étape de transfert une opération de contrôle par visiométrie pour s'assurer qu'une dose de poudre absorbante est à l'intérieur de la compresse.

L'invention sera maintenant plus complètement décrite dans le cadre de caractéristiques préférées et de leurs avantages, en faisant référence aux figures dans lesquelles :
- la figure 1 représente un article à effet refroidissant selon l'invention, dans une vue éclatée où l'on peut voir les deux feuilles formant l'enveloppe de conditionnement, ainsi qu'une compresse et un sachet d'eau qui sont adaptés à être logés entre les deux feuilles dans le produit fini ;
- la figure 2 représente une vue d'ensemble de l'installation de fabrication de l'article représenté à la figure 1, avec une unité de fabrication en série des compresses sèches, des moyens de transfert et une unité de conditionnement sous vide de chaque compresse assemblée avec un sachet d'eau ;
- la figure 3 représente une vue partielle de l'unité de fabrication illustrée à la figure 2, sans les bobines de déroulement de films de matériau non tissé ;
- les figures 4 et 5 sont des agrandissements des zones IV et V visibles sur la figure 3 ;
- la figure 6 représente un agrandissement du premier poste de soudage dans l'unité de fabrication de la compresse de la figure 3, en fonctionnement à vide sans feuilles ;
- la figure 7 représente un agrandissement du poste de dépôt de poudre absorbante dans l'unité de fabrication de la compresse de la figure 3, avec en amont le dispositif de déformation de la feuille inférieure de matériau non tissé et en aval l'arrivée de la feuille supérieure de matériau non tissé.

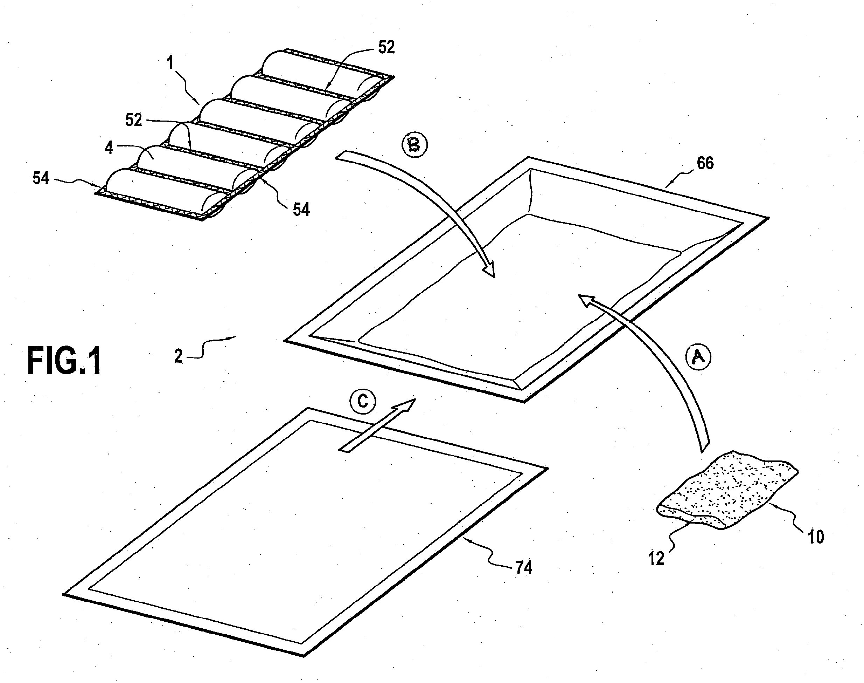

La compresse sèche 1 suivant l'invention est présentée comme partie intégrante d'un article 2, comme cela est illustré pour une meilleure compréhension par une vue éclatée sur la figure 1.

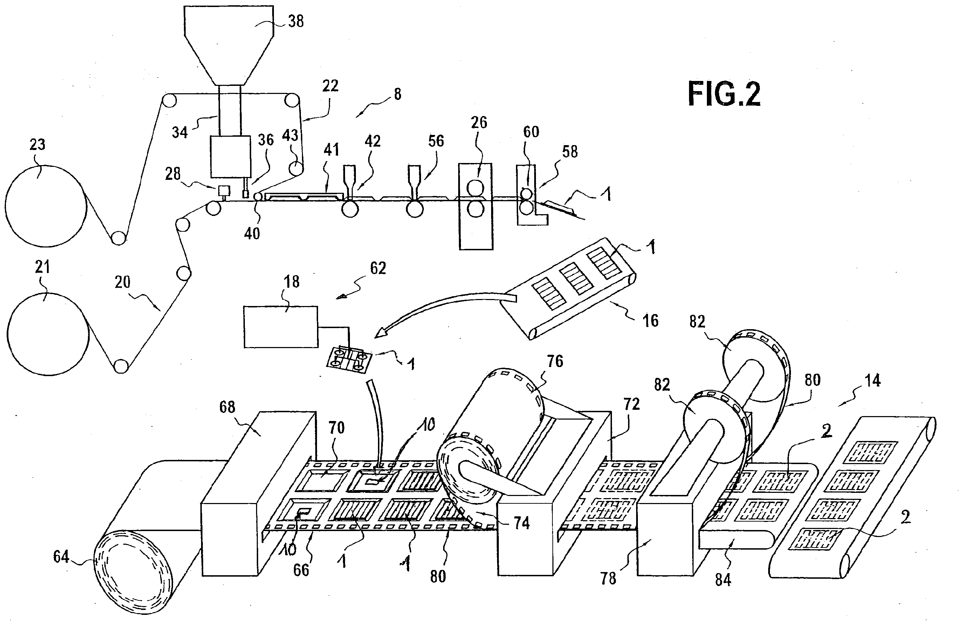

Un article tel que décrit précédemment est fabriqué de la façon suivante, en se référant particulièrement aux figures 2 à 7.

Une compresse sèche est préparée, sous forme par exemple d'un ensemble de compartiments 4 comportant chacun une quantité adéquate de particules 6 de polymère à l'état sec, dans une unité de fabrication de la compresse 8 décrite ci-après.

Un sachet d'eau 10 est préparé indépendamment, dans une unité de fabrication de sachet d'eau, en prenant soin de ménager sur sa surface, et notamment au niveau d'une ligne de soudure, une zone frangible 12.

Une compresse et un sachet d'eau sont alors disposés dans l'unité de conditionnement de l'article 14, dans lequel les feuilles inférieure et supérieure de l'enveloppe de conditionnement sont collées pour enfermer à l'intérieur de l'enveloppe la compresse et le sachet.

L'article 2 est alors soumis à un traitement de stérilisation par rayons gamma ou par rayons bêta. L'intérieur de l'enveloppe, dont la compresse et l'eau du sachet, est ainsi rendu stérile. Aucun des éléments inclus dans le sachet ne subit de dommage, à l'exception d'une partie des particules dont la réticulation est détruite. Il reste cependant encore suffisamment de particules opérantes pour assurer la délivrance d'un froid efficace.

On va maintenant décrire plus en détail le procédé de fabrication selon l'invention et l'installation de fabrication permettant la mise en œuvre de ce procédé, en se référant en particulier où l'installation comporte deux unités de laminage en ligne sur le même site, à savoir une unité de fabrication de compresses sèches 8 et une unité de conditionnement des compresses en articles finis 14, avec un tapis convoyeur 16 et des préhenseurs 18 pour réaliser le transfert des compresses sèches d'une unité vers l'autre.

Les compresses sèches fabriquées sont destinées à alimenter, associées avec des sachets d'eau, l'unité de conditionnement. Elles sont prises en sortie de l'unité de fabrication des compresses sèches elles-mêmes et elles sont disposées par le côté de l'unité de conditionnement, alors qu'un film a déjà été préformé dans ladite unité de conditionnement pour former des barquettes de réception de ces compresses et sachets.

L'unité de fabrication des compresses sèches 8 comporte deux rouleaux pour dévider en continu des feuilles de matière textile perméables à l'eau 20, 22, qui vont être amenées dans cette installation à être soudées l'une à l'autre après que de la poudre absorbante 6 ait été injectée entre ces feuilles, un poste de découpe permettant à la suite des opérations de soudure de scinder les bandes en des compresses sèches,

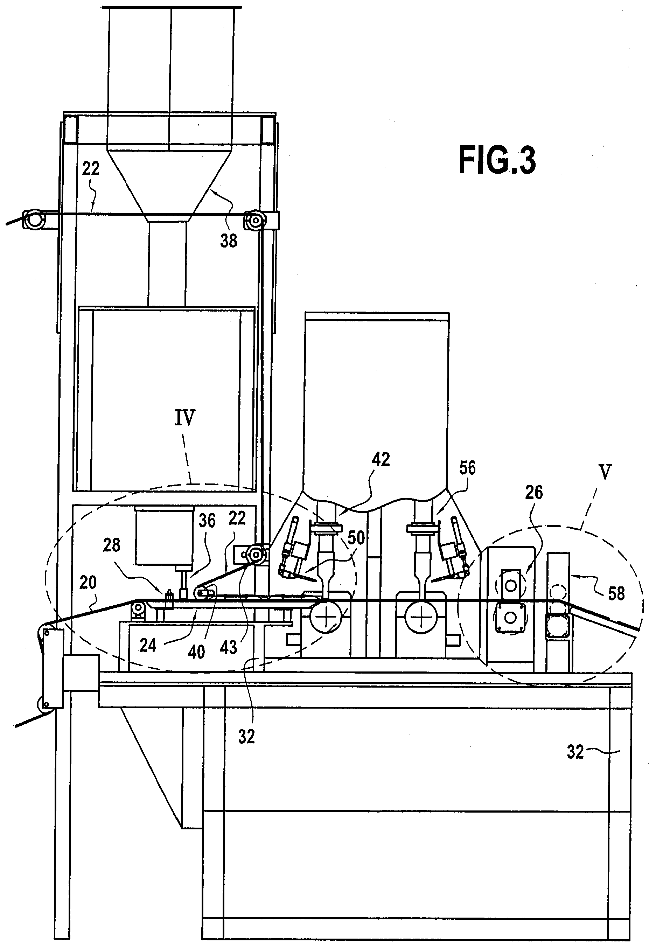

A partir d'un premier rouleau de film 21 on déroule en continu le film de matière textile qui est adapté à former la feuille inférieure de la compresse 20. Le film dévidé est repris par des rouleaux de renvoi jusqu'à être amené en regard d'un tapis de convoyage 24 (visible à la figure 3). Le film est entraîné par traction en bout de l'installation par des rouleaux d'entraînement 26, tel que cela sera décrit ci-après.

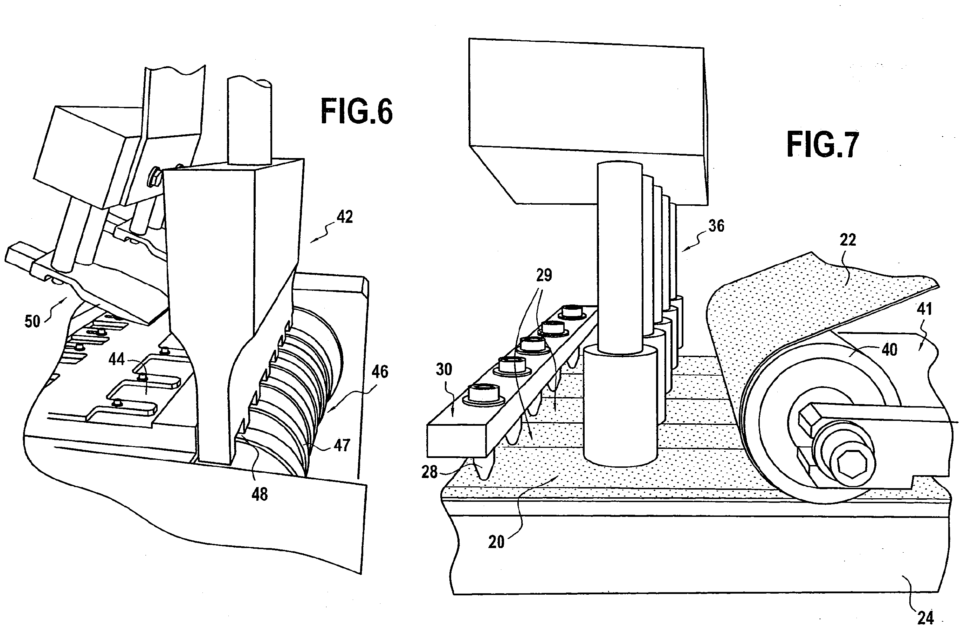

Des doigts de préformage 28 sont disposés au-dessus du tapis, sur le passage de la feuille inférieure. Ils sont en saillie du plan de défilement de la feuille inférieure et ils créent une déformation élastique temporaire de la feuille inférieure en forme de sillons longitudinaux 29. La déformation de la matière textile est réalisée thermiquement par les doigts qui chauffent et creusent des sillons continus le long de la feuille inférieure en défilement. Les doigts sont portés par un bras 30 (figure 7) fixé au bâti de l'installation 32 et qui passe au-dessus et de part et d'autre de la feuille inférieure en défilement et du tapis de convoyage. On déforme mécaniquement la feuille inférieure vers le bas pour creuser par déformation élastique des sillons qui vont former par la suite, lorsque la compresse sèche sera fabriquée, les compartiments de rétention de la poudre.

On trouve en aval de ces doigts de préformage, immédiatement après dans le sens de défilement de la bande, un dispositif de dépôt de poudre 34 comportant un support de doseurs 36 montés en sortie d'une trémie d'alimentation en poudre de particules absorbantes 38. Les doseurs sont espacés l'un par rapport à l'autre pour que chaque doseur 36 soit situé au-dessus des sillons formés précédemment dans la feuille en défilement.

Le distributeur est du type à cylindre rotatif à la surface duquel sont creusées des alvéoles d'entraînement de la poudre dirigées vers les doseurs. Dans le cas où l'unité de fabrication est à défilement de la gauche vers la droite, comme illustré sur les figures, une hotte d'aspiration des particules en excès, ici non représentée, est prévue à gauche du cylindre rotatif distributeur.

Périodiquement, au rythme auquel se succèdent les compresses à distinguer les unes des autres, chaque doseur 36 délivre une dose prédéterminée de poudre absorbante 6 dans chaque sillon 29.

Dans chaque sillon, la poudre est ainsi déposée, par doses successives, sur une partie seulement de la longueur pour permettre de réaliser par la suite les soudures de fermeture des sillons dans le sens transversal par rapport à la direction de défilement et la découpe des compresses successives entre deux lignes de soudure transversales.

On peut observer que l'on a autant de doigts et de doseurs que l'on souhaite de compartiments de rétention de la poudre, pour une compresse donnée. Dans l'exemple représenté, on a six doigts et six doseurs, pour six sillons, comme cela est particulièrement visible sur la figure 7.

Directement après le dispositif de dépôt, la bande de matière textile, perméable à l'eau elle aussi, qui forme la feuille supérieure 22 dans la compresse est plaquée sur la feuille inférieure 20, en emprisonnant entre les feuilles la poudre déposée préalablement. Du fait de la disposition de la poudre dans les sillons, la poudre n'est pas dispersée lors du placage de la feuille supérieure sur la feuille inférieure.

La feuille supérieure est dévidée depuis un deuxième rouleau 23. On comprendra que le deuxième rouleau pourrait être fixé au bâti de l'installation à proximité de la zone de recoupement des deux feuilles. Toutefois, tel qu'illustré sur les figures, il est avantageux que les deux rouleaux soient disposés à proximité, On a ainsi une seule zone de chargement de tissu et, dans le cas où elle est prévue dans l'installation, une unique zone de raboutage dans laquelle les deux feuilles passent sur des tables de raboutage servant à raccorder bout à bout la bande en fin de rouleau avec le début d'une bande d'un rouleau suivant.

Le trajet de la feuille supérieure est alors plus long en ce que la feuille doit passer au-dessus des doseurs 36 pour qu'elle n'entrave pas l'alimentation en poudre pour la distribution entre la feuille supérieure et la feuille inférieure de la compresse.

La feuille supérieure est plaquée contre la feuille inférieure, et contre la poudre déposée sur cette feuille inférieure, au niveau d'un rouleau de plaquage 40 sur lequel est entraînée la feuille supérieure et sous lequel la feuille inférieure passe. A ce niveau, la feuille inférieure 20 repose sur le tapis de convoyage 24 qui commence en amont des doigts de préformage et qui va jusqu'au premier poste de soudage 42 des deux feuilles l'une sur l'autre. Comme cela sera décrit ci après, le tapis de convoyage est adapté à passer sous une table de soudage 41 au niveau de laquelle les feuilles et la poudre à l'intérieur vont être compressées entre le tapis et ladite table.

On prévoit des rouleaux de renvoi pour le trajet de la feuille supérieure comme pour le trajet de la feuille inférieure, On a ici en particulier le rouleau 43 disposé avant le rouleau de plaquage 40 sur le trajet de la feuille supérieure, qui est monté selon un axe flottant non parallèle à l'axe de rotation des autres rouleaux pour faciliter l'ajustement transversal de la feuille supérieure pour que les bords de celle-ci correspondent avec les bords de la feuille inférieure,

L'ensemble, ainsi formé par les feuilles inférieure et supérieure entre lesquelles repose de la poudre, passe sur le tapis de convoyage et sous la table de soudage qui est directement montée sur le bâti après le rouleau de plaquage et qui permet le maintien en position de l'ensemble tout en l'aplatissant, ce qui assure la dispersion de la poudre à l'intérieur des sillons. La table de soudage présente des plots en saillie 44, disposés sur le parcours des feuilles au niveau des intervalles entre les sillons, où la poudre n'est pas présente. Ces plots en série dans le sens longitudinal maintiennent dans les intervalles entre les sillons la feuille supérieure fermement plaquée contre la feuille inférieure qui est pressée sur le tapis de convoyage. Ceci permet de conserver sous cette table de soudage la répartition de la poudre dans les sillons alors que l'ensemble est aplati et que la creusure des sillons s'élargit, et ceci permet ainsi d'éviter à l'arrivée sur les postes de soudage par ultrasons que de la poudre se retrouve dans ces intervalles, qui correspondent aux futures lignes de soudure longitudinales entre les sillons et qui doivent donc rester vierges de poudre pour un soudage optimisé.

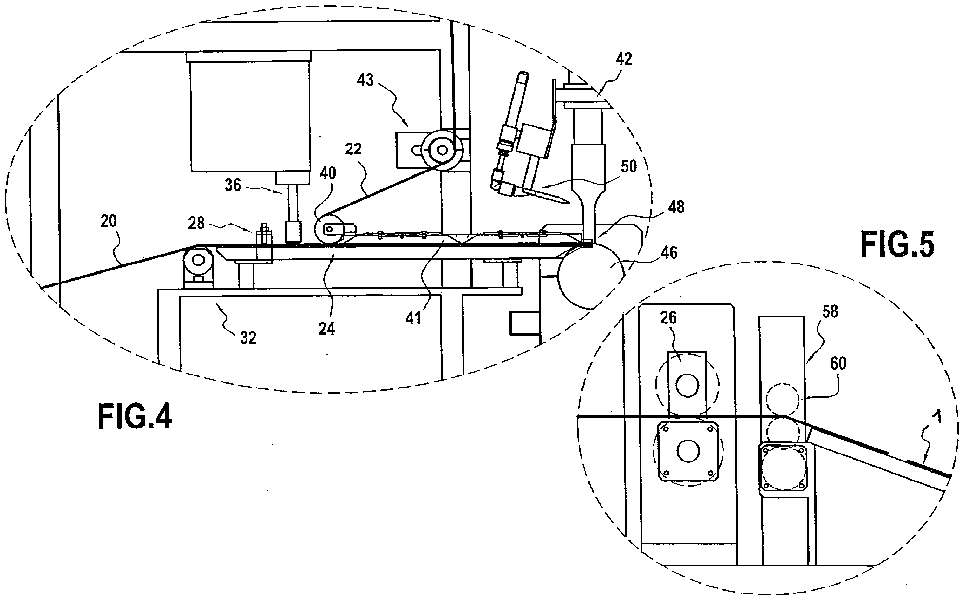

En sortie de la table de soudage, l'ensemble quitte le tapis convoyeur et il passe sur un premier poste de soudage par ultrasons 42 qui comporte une enclume 46 et un ensemble de sonotrodes 48 (figures 4 et 6). L'enclume est formée par un rouleau sur lequel sept bandes de roulement 47 sont disposées en relief et qui correspondent aux cinq intervalles entre les sillons et aux deux bords d'extrémités latérales. On a ainsi sept sonotrodes qui viennent appliquer une pression sur les feuilles au niveau de chacune des bandes de roulement et restituer l'énergie provoquée par les ultrasons pour souder par point la feuille supérieure à la feuille inférieure longitudinalement, dans le sens de défilement de la bande.

Le premier poste de soudage comporte en outre des buses de soufflage d'air de refroidissement 50, comme cela est illustré sur les figures et particulièrement visible sur les figures 4 et 6.

La bande en défilement est alors faite des deux feuilles de matériau non tissé perméables enserrant entre elles les îlots de poudre de particules super absorbantes qui sont étalés et retenus dans les cavités formées par les sillons et qui sont isolées désormais par des lignes de soudure longitudinales 52. On réalise alors dans chaque sillon la soudure de lignes transversales 54 en travers du défilement assurant la séparation des sections successives de film et de poudre destinées à constituer des compresses sèches successives,

L'ensemble passe à cet effet sur un deuxième poste de soudage par ultrasons 56 qui comporte ici une sonotrode et une enclume formée, en vrille sur un cylindre rotatif. L'enclume présente un relief sur une largeur correspondant à deux fois une ligne transversale d'extrémité d'une compresse, étant entendu que cette soudure est réalisée avant découpe. On a une sonotrode qui vient appliquer une pression sur les feuilles au niveau de la bande de roulement et restituer l'énergie provoquée par l'ultrason pour souder la feuille supérieure à la feuille inférieure transversalement.

Comme le premier poste de soudage, le deuxième poste de soudage comporte un dispositif de refroidissement du cylindre d'enclume. Avantageusement, ce dispositif peut comporter un baffle en courbe sur lequel sont dirigés les jets des buses de soufflage d'air de refroidissement. On évite ainsi que ces jets puissent atteindre directement les emplacements contenant les particules de poudre et qu'ils puissent en déplacer vers les plages réservées aux soudures.

Le déplacement de la bande en défilement, désormais constituée de compresses sèches successives mais toujours accrochées les unes aux autres transversalement avec chaque compresse qui s'étend sur toute la largeur de la bande, se poursuit sous l'effet d'un mécanisme à deux rouleaux d'entraînement 26 tournant en sens inverse et qui pincent entre eux les compresses successives. Ces rouleaux d'entraînement assurent la traction nécessaire à l'entraînement des bandes à travers les différents dispositifs de l'unité de fabrication disposés en amont. Le mouvement donné à la bande l'entraîne vers le dispositif de découpe 58 situé en aval de ces rouleaux d'entraînement.

Le dispositif de découpe comporte deux rouleaux entre lesquels passent les compresses sèches successives. Un des rouleaux possède une lame tranchante 60 disposée en hélice et qui à chaque passage en regard du rouleau opposé vient couper l'ensemble transversalement. La vitesse de révolution de ce rouleau à lame tranchante ainsi que son diamètre sont tels que la lame vient couper transversalement l'ensemble en défilement au milieu de chaque soudure transversale d'extrémité, en séparant ainsi les unes des autres successivement chaque compresse sèche fabriquée.

La compresse sèche découpée 1 est placée en sortie de l'unité de fabrication 8 sur le tapis convoyeur 16.

Comme cela est illustré sur la figure 2, l'installation comporte un dispositif de transfert à préhension pneumatique 62 qui est piloté pour saisir automatiquement un groupe de compresses sèches disposées sur le tapis convoyeur, les transférer depuis ladite unité de fabrication de compresses vers l'unité de conditionnement et les libérer par désactivation des ventouses pour les déposer dans leurs emplacements respectifs, c'est à dire tel que cela sera décrit ci-après sur la feuille d'enveloppe inférieure de l'article qui a déjà été préformée.

Chaque préhenseur 18 du dispositif de transfert 62 comporte une ventouse et des moyens de liaison de cette ventouse à un circuit de vide. Chacun des préhenseurs est ainsi apte respectivement à saisir une compresse sèche par aspiration au moyen de sa ventouse, et à libérer une compresse sèche saisie par soufflage d'air dans ladite ventouse. Les préhenseurs sont groupés en modules formant ensemble une matrice de préhension. Les ventouses des différents préhenseurs forment un maillage en deux dimensions d'une surface d'aire prédéterminée.

Avantageusement, le dispositif de transfert 62 est adapté à saisir simultanément plusieurs compresses sur le tapis de convoyage, ici deux pour pouvoir venir les disposer en même temps dans des barquettes formées côte à côte dans l'unité de conditionnement. On comprend que l'essentiel selon l'invention pour cette opération de transfert est que les compresses sèches et les sachets d'eau associés soient positionnés dans la barquette correspondante avant que la feuille supérieure soit rabattue pour les enfermer sous conditionnement sous vide.

Le dispositif de transfert peut être associé à un poste de contrôle qui s'assure par visiométrie que la compresse sèche contrôlée, avant remplissage de l'article stérile, comporte bien de la poudre absorbante.

Comme on l'a indiqué plus haut, l'installation selon l'invention qui est décrite ici comporte en outre une unité de conditionnement sous vide en articles finis 14 dans laquelle on opère également par laminage, à partir de deux rouleaux d'où sont dévidés des films de matériau plastique imperméable, qui vont être collés thermiquement l'un à l'autre pour former une enveloppe de conditionnement après qu'une compresse et qu'un sachet d'eau aient été placés entre les deux films, dans une barquette préformée dans l'un des films, et après que le vide y ait été fait.

Un premier rouleau 64 d'un film étanche à l'eau et à l'air se déroule et le film est adapté à former la feuille inférieure de l'article 66, qui alimente en continu l'unité de conditionnement sous vide.

La feuille inférieure passe dans un four 68, dans lequel on vient réaliser un formage thermique des zones de la feuille, pour former ainsi des barquettes de réception 70. Ici, deux barquettes sont formées côte à côte sur une même largeur de feuille.

Dans le mode de réalisation représenté, la feuille inférieure est maintenue tendue entre deux séries de pinces qui saisissent des bords et qui sont portées par deux chaînes latérales d'entraînement de la feuille, de sorte qu'il n'est pas prévu de contre moule pour le préformage.

Les chaînes latérales d'entraînement assurent un défilement en saccadé de la feuille inférieure et par la suite, comme cela sera décrit ci-après, celui de la feuille supérieure. Cet entraînement en saccadé, distinct du défilement continu dans l'unité de fabrication des compresses sèches, permet ici d'assurer un temps de pause nécessaire pour que la feuille inférieure reste suffisamment de temps dans le four pour réaliser le formage voulu puis, comme cela va être décrit, la mise sous vide et le soudage thermique,

En sortie du four de formage, les barquettes 70 sont remplies par une compresse sèche 1 et un sachet d'eau 10. On observe ici que l'arrivée des compresses et des sachets d'eau sur l'unité de conditionnement 14 se fait sur le côté et non en début de poste. Une compresse sèche et un sachet d'eau doivent être logés dans une barquette déjà préformée dans la feuille inférieure de l'enveloppe de conditionnement, avant d'être recouverts par la feuille supérieure de cette enveloppe. On comprend alors que l'épaisseur des compresses en sortie de l'unité de fabrication est déterminée pour qu'une compresse sèche et le sachet d'eau associé soient entièrement contenus dans le volume de la barquette.

Les compresses sèches sont transférées par les préhenseurs 18. Les sachets d'eau peuvent l'être également, à moins qu'ils sont apportés manuellement. Comme cela sera décrit ci après en variante, la disposition du sachet d'eau par rapport à la compresse dans l'article peut être différente de celle représentée sur les figures 1 et 2, dans laquelle le sachet d'eau est déposé initialement contre la feuille inférieure de l'enveloppe de conditionnement (comme cela est représenté par la flèche A de la figure 1), la compresse étant déposée sur le sachet d'eau de sorte que celui-ci repose entre la compresse et la feuille inférieure de l'enveloppe.

Dans ce mode de réalisation, la compresse est placée au-dessus du sachet d'eau (flèche B, figure 1) et est ensuite maintenue dans cette position par le rabat de la feuille supérieure (flèche C, figure 1). On place le sachet d'eau initialement au centre de la barquette et on pose la compresse par dessus sans modifier cette position centrale du sachet d'eau. Il peut avantageusement être prévu un moyen adhésif sur le sachet d'eau pour lui faire garder cette position centrale qui est préférée pour des raisons d'écoulement d'eau homogène dans la compresse.

On observe qu'il est particulièrement intéressant de disposer le sachet d'eau contre la feuille inférieure de la compresse dans le cas où celle-ci est plus perméable à l'eau que la feuille supérieure de la compresse.

La feuille inférieure de l'enveloppe, avec la compresse sèche et le sachet d'eau associés disposés dans les barquettes, passe ensuite dans un poste de soudage thermique et de mise sous vide 72. Préalablement, une feuille supérieure 74 est plaquée au-dessus de la feuille inférieure. A cet effet, un rouleau 76 est fixé au-dessus du poste de soudage thermique et se déroule en continu.

La feuille supérieure est également tendue entre les deux séries de pinces qui saisissent des bords et qui sont portées par deux chaînes latérales d'entraînement de la bande. La tension entre la même série de pinces permet l'ajustement transversal de la feuille supérieure par rapport à la feuille inférieure et le maintien en tension de ces feuilles nécessaire pour le soudage et la mise sous vide sans qu'il y ait de rétractation.

Le soudage et la mise sous vide dans le poste 72 sont réalisés en trois étapes, tout d'abord en fermant les bords autour de chaque barquette par soudure des feuilles l'une sur l'autre à l'exception du bord arrière de ladite barquette, puis en faisant le vide successivement dans le sens de défilement de l'ensemble en amont de la soudure transversale du bord arrière de chaque barquette, avant enfin de procéder à la fermeture complète de chaque barquette par soudure de ce bord arrière laissé ouvert initialement pour la mise sous vide. Cette opération de soudage thermique se fait donc sur toutes les plages entourant les barquettes réceptrices des compresses. Le poste de soudage thermique assure ainsi la fixation de la feuille supérieure contre la feuille inférieure, enfermant de fait la compresse et le sachet d'eau à l'intérieur dans un environnement sous vide étanche.

Les matériaux des feuilles d'une même enveloppe de conditionnement sont de nature différente suivant qu'il s'agit de la feuille supérieure ou de la feuille inférieure qui doit être réceptrice à l'opération de formage thermique des barquettes. Par exemple, la feuille inférieure doit être formée d'un matériau thermodurcissable pour prendre à demeure la forme en barquette tandis que la feuille supérieure peut être dans un matériau plastique autre. Toutefois, les deux feuilles sont étanches à l'eau, principalement pour retenir l'eau à l'intérieur lorsque le sachet d'eau est crevé. Elles sont également étanches à l'air pour que l'ensemble reste sous vide et préserve son état stérile en cas de traitement préalable, à l'abri de toute intrusion des bactéries de l'air ambiant. Les deux feuilles sont soudées thermiquement sur leurs faces en regard pour assurer cette étanchéité et ce vide. En outre, une ligne de jonction, avantageusement la soudure du bord arrière réalisée après que le vide ait été fait, est une ligne à faible résistance pour permettre la pelabilité et assurer ainsi manuellement l'ouverture du dispositif médical fini en vue de l'utilisation de la compresse imbibée d'eau et refroidie qu'il contient. Cette pelabilité est facilitée par la réalisation d'au moins l'une des deux feuilles de l'enveloppe avec une couche extérieure de matériau faiblement adhésif.

Comme précédemment, les chaînes latérales d'entraînement assurent un défilement en saccadé des deux feuilles qui permet d'assurer un temps de pause nécessaire pour que l'ensemble reste un temps suffisant dans le poste de soudage et de mise sous vide pour que le vide soit réalisé et que les feuilles soient soudées l'une à l'autre.

En sortie de ce poste, l'ensemble est repris sur les bords des feuilles soudées l'une sur l'autre par des pinces prévues sur des chaînes transporteuses comme précédemment, assurant un même défilement en saccadé.

L'ensemble passe alors dans un poste de découpe 78 dans lequel le découpage des compresses sous emballage individuel est réalisé à l'emporte pièce, avec une coupe longitudinale et transversale pour séparer les articles l'un de l'autre. On observe qu'une découpe est aussi faite sur les côtés. Les extrémités latérales des films 80 sont alors enroulés sur des bobines 82 fixées en sortie du poste de découpe et prévues de part et d'autre du tapis, et qui réalisent l'entraînement des feuilles à travers l'ensemble de l'unité de conditionnement par bobinage des bords.

Lors de la découpe, les articles sous enveloppe de conditionnement tombent. Ils peuvent tomber directement dans un bac ou sur un tapis de sortie 84 qui prend forme dans le poste de découpe.

Il est ensuite prévu de ranger les articles côte à côte dans des cartons dans une disposition verticale pour éviter le risque d'ouverture prématuré des sachets d'eau qui pourrait se produire dans le cas d'un empilement des dispositifs à plat horizontalement les uns sur les autres.

Comme cela a été décrit précédemment, les sachets d'eau disposés dans les barquettes avec les compresses sèches peuvent être amenés manuellement ou être transférés par un dispositif de transfert équivalent à celui décrit précédemment depuis une machine de fabrication des sachets d'eau que peut comporter avantageusement l'installation de fabrication selon l'invention.

La machine est du même type que celles qui sont utilisées pour toutes sortes de fabrication de berlingots de liquide. Une feuille en matière plastique est pliée de sorte que les deux bords latéraux se superposent pour former un tube fermé sur lui-même par soudure le long d'une génératrice. Cette soudure latérale est réalisée de manière à former par la suite une zone frangible adaptée à céder quand l'utilisateur appuie fortement sur l'article pour imbiber d'eau la compresse sèche afin d'activer son effet refroidissant sur une plaie.

Une fois cette soudure latérale effectuée, on place le tube dans des mâchoires de soudure thermique en transversal de l'axe du tube et on réalise une soudure transversale du tube qui va former la ligne de soudure inférieure du sachet. On fait ensuite s'écouler de l'eau sous pression par doses successives au centre du tube de sorte que le tube se remplisse d'eau, tout en vérifiant le niveau de remplissage par une sonde, L'eau est filtrée sur des tamis à 0,2 microns et elle est en plus additionnée de chlore pour un traitement antibactérien évitant sa contamination dans les tuyaux qui la véhiculent. Lorsque le niveau d'eau a atteint un seuil prédéterminé, on réalise une soudure transversale supérieure en formant alors un sachet qui enferme l'eau. On fait attention à ce que cette ligne de soudure supérieure soit faite suffisamment en dessous du niveau de l'eau, Ainsi lors de la soudure en chassant l'eau en excédent, on s'assure d'une part que l'on n'enferme pas des bulles d'air dans le sachet et d'autre part que la quantité d'eau présente dans le sachet est bien la quantité requise, nécessaire à la bonne imprégnation ultérieure de la compresse. On peut noter accessoirement qu'au cas où une minime bulle d'air serait enfermée dans le liquide, elle en serait nécessairement évacuée lors de la mise sous vide de l'ensemble formé par l'enveloppe la compresse et le sachet, dans la mesure où les parois du sachet sont en un matériau polymère à base de polyéthylène et où elles sont de ce fait perméables à l'air,

Dans une production de sachets d'eau en continu, le sachet ainsi formé est découpé et dégagé de l'installation pour être par la suite disposé dans l'enveloppe de conditionnement. La ligne de soudure supérieure du sachet précédent forme alors la ligne de soudure inférieure du sachet suivant et le processus de remplissage, de soudure et de découpe se poursuit cycliquement.

La soudure latérale est réalisée pour d'une part assurer la fermeture étanche à l'eau du sachet pendant tout le temps des opérations de fabrication et résister aux manipulations des sachets et d'autre part pour céder, dans le produit fini, dès que l'on appuie à plat sur l'article et donc sur le sachet d'eau, afin que l'eau vienne imbiber la compresse.

On va maintenant décrire des variantes de l'installation non représentées, et dans lesquelles on prévoit différemment les moyens de retenue en position du sachet d'eau par rapport à la compresse.

Dans une première variante, le sachet d'eau est disposé en premier dans le fond de la barquette avant la compresse sèche, comme décrit précédemment, et cette barquette comporte elle-même une cuvette préformée au fond, qui s'étend au centre et qui est de la forme du sachet d'eau. Le sachet est glissé dans la cuvette et y est bloqué en translation dans deux directions. La mise en position de la compresse sèche dans la barquette enferme définitivement le sachet qui ne peut plus bouger La cuvette étant placée au centre de la barquette, le sachet d'eau prend une position centrale par rapport à la compresse sèche, qui ne bougera pas lorsque la feuille supérieure est rabattue sur la feuille inférieure de l'enveloppe. Là aussi, l'enveloppe de conditionnement est suffisamment volumineuse pour contenir la compresse sèche et le sachet d'eau posés à plat l'un sur l'autre.

Dans une deuxième variante, c'est la compresse qui est disposée en premier dans le fond de la barquette, et on rapporte le sachet d'eau sur la compresse, en son centre. On peut alors prévoir de retourner dessus-dessous la compresse sèche à l'occasion de son transfert par les préhenseurs, et de disposer la compresse sèche en premier dans l'enveloppe de conditionnement avec la feuille inférieure qui est tournée vers le haut, prête à recevoir le sachet d'eau. Ainsi, le sachet d'eau se trouve directement au contact de la feuille inférieure et l'eau qui est expulsé du sachet est plus rapidement au contact des particules super absorbantes. Le sachet d'eau est maintenu dans une position centrale par le rabat immédiat de la feuille supérieure à plat qui bloque le sachet par rapport à la compresse.

Dans les deux cas, on s'assure comme dans le mode de réalisation décrit précédemment de la bonne position du sachet d'eau par rapport à la compresse sèche pour une meilleure diffusion de l'eau au moment de l'ouverture du sachet d'eau.

Il résulte de ce procédé de fabrication une compresse facile à utiliser et répondant aux critères exigeants de stérilité des milieux hospitaliers.

L'article comporte, à l'intérieur d'une enveloppe de conditionnement sous vide formée de deux feuilles 66, 74 soudées l'une sur l'autre, un sachet 10 rempli d'eau et fermé étanche à l'eau par une paroi comportant une zone frangible 12 ainsi que la compresse 2 qui renferme des particules de polymère à l'état sec 6. L'enveloppe de conditionnement est constituée en une matière étanche à l'eau et à l'air de nature à maintenir le sachet d'eau et la compresse à l'état stérile sous vide.

Les feuilles formant la paroi de l'enveloppe de conditionnement sont par exemple constituées d'un mélange de polyamide et de polyéthylène. Un tel matériau a la particularité d'être compatible avec un traitement de stérilisation par rayons gamma. Ceci signifie que d'une part, ce matériau n'est pas dégradé lors d'un tel traitement de stérilisation, et que d'autre part il laisse passer les rayons gamma, permettant ainsi de réaliser une stérilisation efficace des éléments contenus dans l'enveloppe de conditionnement.

Avantageusement, les parois de l'enveloppe de conditionnement sont transparentes, si bien qu'on voit à travers elles. Ceci permet d'être à même de surveiller l'état des éléments contenus dans l'enveloppe de conditionnement.

A l'intérieur de l'enveloppe de conditionnement, sont disposés la compresse et le sachet rempli d'eau.

La compresse comporte deux feuilles soudées l'une à l'autre et entre lesquelles sont disposées des particules d'un polymère à forte capacité d'absorption d'eau, initialement à l'état sec.

Les feuilles qui renferment les particules sont constituées en matériau non-tissé perméable à l'eau et à l'air. Ce matériau est compatible avec un traitement de stérilisation par rayons gamma, ce qui implique que d'une part il résiste à un tel traitement, et d'autre part il ne bloque pas le passage des rayons gamma, permettant ainsi la stérilisation des particules qui se trouvent à l'intérieur de la compresse.

De plus, le matériau des feuilles présente une bonne résistance à la pression, celle qui est exercée par l'air ambiant sur l'article sous vide avant l'activation du polymère, celle aussi qui est exercée sur les parois de la compresse par les particules de polymère lorsque celles-ci gonflent par absorption d'eau, en se dilatent beaucoup et de façon rapide. Par ailleurs, les feuilles sont meilleurs en perméabilité à l'eau pour activer le polymère à effet refroidissant si elles sont réalisées en une matière non absorbante pour l'eau. A cet effet, on utilise de préférence des fils de résine artificielle, comme de la résine de polyester.

Sur ses bords formés par les extrémités en vis à vis de chaque paroi, la compresse est fermée par des lignes de soudure afin d'éviter que les particules ne s'en échappent. Les lignes de soudure sont relativement larges, notamment de largeur comprise entre 3 et 7 mm, ce qui augmente leur résistance à l'effort exercé sur elles par les particules de polymère qui se dilatent lorsqu'elles absorbent de l'eau.

Les feuilles formant la compresse sont adhérentes l'une à l'autre suivant différentes lignes longitudinales réalisées par soudure par ultrasons, de manière à former une pluralité de compartiments allongés dans lesquels sont réparties de manière homogène les particules de polymère. Dans l'exemple de réalisation représenté sur les figures et tel que cela est particulièrement visible sur la figure 1, la compresse comporte six compartiments isolés l'un de l'autre par rapport aux particules de polymère. Les lignes de soudure obtenues sont en outre mécaniquement résistantes aux efforts exercés sur elles par les particules de polymère gonflant par absorption d'eau.

Les particules de polymère absorbant utilisées sont en elles-mêmes connues, telles qu'elles sont choisies ici pour être dépourvues de toxicité. Ces particules de polymère, qui sont à l'origine de l'effet de refroidissement produit par la compresse, quand les particules qui ont été préalablement activées, c'est-à-dire gonflées par de l'eau qu'elles ont absorbée, après éventuellement avoir subi un séjour dans un environnement froid, génèrent du froid par désorption et vaporisation de l'eau qu'elles avaient absorbée.

Le sachet d'eau est fermé de façon hermétique, notamment par soudure. Les soudures réalisées sont suffisamment résistantes pour ne pas céder lorsque l'article est manipulé pour son transport. Cependant, l'une au moins des soudures est conçue de telle sorte qu'elle puisse céder, au moins en partie, sous l'effet d'une pression qui serait exercée par l'utilisateur sur la surface extérieure du sachet d'eau, à travers l'enveloppe de conditionnement. Ainsi, lorsque l'on souhaite que l'eau contenue dans le sachet d'eau se répande à l'extérieur de ce sachet, une pression assez forte exercée avec la main sur la compresse permet de faire rompre la zone frangible du sachet et de libérer l'eau pour quelle vienne à l'intérieur de l'enveloppe de conditionnement, imprégner le polymère retenu dans l'enveloppe de la compresse proprement dite.

Le sachet d'eau contient uniquement la quantité d'eau adéquate, autant que possible sans bulles d'air. Cette quantité est déterminée en fonction de la quantité de particules de polymère incluse dans les différents compartiments de rétention de la poudre de polymère et de la capacité d'absorption d'eau des particules, de manière à ce qu'elle soit entièrement absorbée lors de l'ouverture du sachet d'eau. Il est doublement avantageux que la quantité de polymère soit en excès par rapport à l'eau, d'une part pour éviter qu'il subsiste de l'eau liquide dans l'enveloppe de conditionnement lors de son ouverture et d'autre part pour que les particules restent en demande d'eau au voisinage des parois en matière textile de la compresse.

## Revendications

1. Procédé de fabrication d'articles à compresses à effet refroidissant contenant des particules de polymère absorbant, consistant à procéder par laminage entre deux feuilles (20,22) de rétention desdites particules qui sont entraînées en défilement continu le long d'une unité (8) de fabrication de compresses sèches dans laquelle, successivement : on ménage des sillons (29) dans celles des feuilles qui se présente en feuille inférieure (20) en provoquant sa déformation thermique réversible par l'application de doigts chauffants (28) se disposant en ligne en travers de la direction de défilement, on dépose dans chacun desdits sillons des doses successives de polymère absorbant (36) en poudre s'étendant sur une longueur déterminée desdits sillons, on recouvre la feuille inférieure ainsi chargée par l'autre desdites feuilles venant en feuille supérieure, (22) on soude (42) les deux feuilles l'une à l'autre le long des intervalles entre sillons adjacents afin de fermer latéralement des compartiments remplis de polymère absorbant, on les soude (56) ensemble également en bout de chacun desdits compartiments, suivant des lignes de soudure transversales à la direction de défilement là où les sillons sont vides de polymère entre les doses successives déposées, puis on découpe (60) l'ensemble en compresses sèches successives à l'endroit desdites lignes de soudure.

2. Procédé suivant la revendication 1, comportant une étape ultérieure de conditionnement sous vide desdites compresses sèches suivant laquelle on opère par laminage entre deux films en matière étanche à l'eau et à l'air, comportant un film inférieur dans lequel on ménage par déformation plastique à chaud des barquettes dans chacune desquelles on dépose une compresse avec un sachet d'eau associé, contenant une quantité d'eau déterminée pour son activation par gonflement à l'eau de toutes les particules de polymère absorbant qu'elle contient, disposés l'un au-dessus de l'autre, puis on soude les feuilles entre elles entre les barquettes ainsi remplies suivant des lignes de soudure longitudinales et suivant des lignes de soudure transversales tout en faisant le vide entre deux opérations de soudure transversale successives et en découpant l'ensemble suivant lesdites lignes de soudure longitudinales et lesdites lignes de soudure transversales pour séparer les articles obtenus les uns des autres.

3. Installation pour la mise en œuvre du procédé de fabrication selon la revendication 1 ou 2, comportant une unité (8) de fabrication en série dans laquelle on prévoit :
des moyens pour dérouler (21) de manière continue une feuille inférieure de matière textile (20) perméable à l'eau et pour l'entraîner à plat vers la sortie de l'unité en la faisant passer sous une trémie d'alimentation (38) en poudre de particules absorbantes (38) ;
des doigts de préformage (28), disposés en ligne en travers de la direction longitudinale de défilement de la feuille inférieure, pour déformer ladite feuille inférieure en des sillons (29) formés longitudinalement, lesdits doigts chauffant la feuille inférieure tendue et la déformant mécaniquement, de façon thermiquement réversible, les sillons étant aptes à recevoir des doses successives de ladite poudre (6) ;
des moyens pour recouvrir (23,40,43) l'ensemble d'une feuille supérieure (22) qui est déroulée de manière continue et des moyens pour entraîner l'ensemble ainsi formé par laminage vers la sortie de l'unité ;
des premiers moyens de soudure (42) aptes à souder ensemble la feuille supérieure et la feuille inférieure le long des intervalles entre sillons adjacents, de manière à isoler transversalement les uns des autres les sillons adjacents remplis de poudre ;
des seconds moyens de soudage (56) aptes à souder l'une avec l'autre lesdites feuilles suivant des lignes de soudure transversales (54) en travers de l'ensemble en défilement, entre les zones de dépôt des doses successives de poudre, de manière à fermer dans chaque sillon des compartiments successifs (4) contenant chacun une dose de ladite poudre de particules absorbantes ;
des moyens de découpe (60) de l'ensemble en compresses sèches (1) successives par passage entre des rouleaux dont l'un porte une lame tranchante (60).

4. Installation selon la revendication précédente, dans laquelle le dépôt de la poudre (6) est réalisé par un ensemble de doseurs (36) espacés l'un par rapport à l'autre pour que chaque doseur soit situé au-dessus de l'un des sillons (29) de la feuille inférieure (20) en défilement.

5. Installation selon la revendication 3 ou 4, dans laquelle on prévoit des moyens de refroidissement à buses de soufflage d'air (50) pour refroidir les zones de soudure, lesdits moyens de refroidissement étant munis de moyens déflecteurs pour protéger les sillons remplis de poudre (6) des jets d'air soufflés.

6. Installation selon l'une des revendications 3 à 5, comportant en outre une unité de conditionnement sous vide (14) disposée en aval de l'unité de fabrication de compresses sèches (8), ladite unité de conditionnement sous vide comportant des moyens pour successivement :
- réaliser par formage thermique des barquettes individuelles (70) pour la réception des compresses sèches dans une première feuille en matière étanche à l'eau et à l'air (66) qui est entraînée à plat vers la sortie de l'unité ;
- déposer dans chaque barquette un sachet d'eau (10) et une compresse sèche (1) l'un par-dessus l'autre ;
- recouvrir l'ensemble par une deuxième feuille en matière étanche à l'eau et à l'air (74) qui est entraînée à plat simultanément avec la première feuille vers la sortie de l'unité ;
- fermer les bords autour de chaque barquette par soudure des feuilles l'une sur l'autre à l'exception du bord arrière de ladite barquette ;
- faire le vide successivement dans le sens de défilement de l'ensemble en amont de la soudure transversale du bord arrière de chaque barquette, avant de procéder à la fermeture complète de chaque barquette par soudure dudit bord arrière ;
- puis on découpe l'ensemble à l'emporte pièces pour obtenir à l'unité les compresses à effet refroidissant conditionnées sous vide.

7. Installation selon la revendication 6, dans laquelle la réalisation par formage thermique des barquettes (70) est dimensionnée pour qu'une compresse sèche (1) et un sachet d'eau (10) soient entièrement contenus dans le volume de ladite barquette.

8. Installation selon la revendication 6 ou 7, comportant des moyens de maintien en position du sachet d'eau (10) pour assurer une position centrale dudit sachet par rapport à la compresse sèche (1) dans l'enveloppe de conditionnement.

9. Installation selon l'une des revendications 6 à 8, comportant en outre une unité de fabrication de sachets d'eau, disposée en amont d'une unité de conditionnement (14), ladite unité de fabrication de sachets d'eau comportant des moyens pour :
- amener et souder l'un contre l'autre les deux bords latéraux d'une feuille en matière plastique pour former un tube fermé sur lui-même par soudure le long d'une génératrice, cette soudure étant réalisée pour d'une part assurer la fermeture étanche du sachet d'eau (10) et résister aux manipulations ultérieures de ce sachet et d'autre part pour former une zone frangible (12) adaptée à céder, dans l'article fini, dès que l'on appuie à plat sur l'article et donc sur le sachet d'eau, afin que l'eau vienne imbiber la compresse ;
- placer le tube ainsi formé dans des mâchoires de soudure thermique en transversal de l'axe du tube et réaliser une soudure transversale de ce tube qui va former la ligne de soudure inférieure du sachet ;
- faire s'écouler de l'eau sous pression par doses successives au centre du tube de sorte que le tube se remplisse d'eau ;
- vérifier le niveau de remplissage d'eau dans le tube par une sonde sensible au niveau de l'eau ;
- réaliser, lorsque le niveau d'eau a atteint un seuil prédéterminé, la soudure transversale supérieure en formant alors un sachet qui enferme l'eau, la ligne de soudure supérieure étant faite suffisamment en dessous du niveau supérieur de l'eau pour chasser l'eau en excédent et s'assurer que l'on n'enferme pas de bulles d'air dans le sachet ;
- découper les sachets d'eau ainsi formés successivement.

10. Installation selon la revendication 9, comportant en outre des moyens de transfert (62) pour transférer les compresses sèches (1) obtenues en sortie de l'unité de fabrication de compresses sèches (8) à l'entrée de l'unité de conditionnement (14), les moyens de transfert étant adaptés à saisir en outre chaque sachet d'eau (10) en sortie de l'unité de fabrication de sachets pour transférer une compresse et un sachet d'eau associé l'un sur l'autre dans chaque barquette préformée (70) dans l'enveloppe de conditionnement.

11. Installation selon la revendication 10 comportant des moyens de contrôle par visiométrie pour s'assurer qu'une dose de poudre de polymère absorbant (6) est présente à l'intérieur de chaque compresse sèche (1) au niveau desdits moyens de transfert (62).

12. Article à compresses à effet refroidissant, (2) obtenu par la mise en œuvre du procédé de fabrication selon la revendication 4 2, comportant en conditionnement stérile dans une enveloppe (66,74) sous vide une compresse (1) à effet refroidissant par désorption d'eau contenant des particules de polymère absorbant à l'état sec et une réserve d'eau (10) d'activation associée dans un sachet étanche, **caractérisé en ce que** ladite compresse sèche se présente à plat entre deux feuilles (20,22) de matière textile de rétention des particules de polymère absorbant, assemblées par laminage, dont l'une est choisie pour être perméable à l'eau et déformable thermiquement de manière réversible qui tend à revenir à sa forme d'origine et dont l'autre est choisie pour présenter de bonnes propriétés mécaniques, notamment en résistance aux efforts de traction.

13. Article suivant la revendication 12 dans lequel ladite enveloppe de conditionnement sous vide est constituée à partir de deux films (66,74) en matériau étanche à l'eau et à l'air qui sont assemblés par laminage et soudés étanches l'un sur l'autre sur les bords de chaque article et dans lequel l'un desdits films, se plaçant avantageusement contre la compresse sèche, est en une matière choisie pour faciliter le préformage de barquettes par déformation permanente à chaud, alors que l'autre desdits films, se plaçant avantageusement contre le sachet d'eau, (10) est en une matière choisie pour présenter de bonnes propriétés mécaniques, assurant d'une part la résistance de l'ensemble au cours de la fabrication par laminage et facilitant d'autre part la fracturation du sachet d'eau par pression manuelle à travers lui.

## Patentansprüche

1. Verfahren zum Herstellen von Kühlkompressen-Artikeln, welche absorbierende Polymerpartikel enthalten, bestehend aus dem Laminieren der Partikel zwischen zwei Rückhaltebahnen (20, 22), die kontinuierlich entlang einer Einheit zum Herstellen von Trockenkompressen (8) bewegt werden, wobei aufeinander folgend:
Rillen (29) in denjenigen der Bahnen hergestellt werden, die als untere Bahn (20) vorkommen, indem ihre reversible thermische Verformung durch die Anwendung von Heizfingern (28) bewirkt wird, die in einer Linie quer zur Laufrichtung angeordnet sind; aufeinanderfolgende Dosen von absorbierendem Polymer (36) in Pulverform, die sich über eine vorbestimmte Länge der Rillen erstrecken, in jede der Rillen eingebracht werden; die so beladene untere Bahn durch die andere der Bahnen, die in Form einer oberen Bahn vorkommt, bedeckt wird (22); die beiden Bahnen entlang der Intervalle zwischen benachbarten Rillen miteinander verschweißt (42) werden, um mit absorbierendem Polymer gefüllte Kompartimente seitlich zu verschließen; diese auch am Ende jedes der Kompartimente entlang von Schweißlinien quer zur Laufrichtung miteinander verschweißt (56) werden, dort, wo die Rillen zwischen aufeinanderfolgenden aufgetragenen Dosen kein Polymer aufweisen, und dann die Anordnung im Bereich der Schweißlinien in aufeinanderfolgende Trockenkompressen geschnitten wird (60).

2. Verfahren nach Anspruch 1, umfassend einen anschließenden Schritt des Vakuumverpackens der Trockenkompressen durch Laminieren zwischen zwei Folien aus wasser- und luftdichtem Material, umfassend eine untere Folie, in der durch Heißplastikverformung Schalen gebildet werden, in die jeweils eine Kompresse mit einem zugehörigen Wasserbeutel abgelegt wird, der eine Wassermenge enthält, die für die Aktivierung durch Aufquellen aller darin enthaltenen, übereinander angeordneten absorbierenden Polymerteilchen mit Wasser bestimmt ist, die Bahnen werden dann zwischen den so gefüllten Schalen entlang von Längsschweißlinien und entlang von Querschweißlinien zusammengeschweißt, wobei zwischen zwei aufeinanderfolgenden Querschweißvorgängen ein Vakuum erzeugt wird, und die Anordnung entlang der Längsschweißlinien und der Querschweißlinien geschnitten wird, um die erhaltenen Artikel voneinander zu trennen.

3. Anlage zur Durchführung des Herstellungsverfahrens nach Anspruch 1 oder 2, umfassend eine Serienfertigungseinheit (8), in der vorgesehen sind:
Mittel zum kontinuierlichen Abwickeln (21) einer unteren Bahn aus wasserdurchlässigem Textilmaterial (20) und zum Flachziehen zum Auslass der Einheit, indem sie unter einem Trichter (38) zum Zuführen von pulverförmigen absorbierenden Partikeln (38) hindurchgeführt wird;
Vorformungsfinger (28), die in einer Linie quer zur Längsrichtung des Laufs der unteren Bahn angeordnet sind, um die untere Bahn in längsgeformte Rillen (29) zu verformen, wobei die Finger die gespannte untere Bahn erwärmen und sie in einer thermisch reversiblen Weise mechanisch verformen, wobei die Rillen dazu geeignet sind, aufeinanderfolgende Dosen des Pulvers (6) aufzunehmen;
Mittel zum Abdecken (23, 40, 43) der Anordnung mit einer oberen Bahn (22), die kontinuierlich abgerollt wird, und Mittel zum Bewegen der so gebildeten Anordnung durch Laminieren zum Auslass der Einheit;
erste Schweißmittel (42), die dazu geeignet sind, die obere Bahn und die untere Bahn entlang der Intervalle zwischen benachbarten Rillen miteinander zu verschweißen, um so benachbarte pulvergefüllte Rillen in Querrichtung voneinander zu isolieren;
zweite Schweißmittel (56), die dazu geeignet sind, die Bahnen entlang von quer verlaufenden Schweißlinien (54) quer durch die laufende Anordnung zwischen den Bereichen der Ablagerung von aufeinanderfolgenden Dosen des Pulvers miteinander zu verschweißen, so dass in jeder Rille aufeinanderfolgende Kompartimente (4) eingeschlossen werden, die jeweils eine Dosis des Pulvers aus absorbierenden Partikeln enthalten;
Mittel zum Schneiden (60) der Anordnung in aufeinanderfolgende Trockenkompressen (1) durch eine Passage zwischen Rollen, von denen eine eine Schneidklinge (60) trägt.

4. Anlage nach dem vorhergehenden Anspruch, wobei die Ablage des Pulvers (6) durch eine Gruppe von Dosiervorrichtungen (36) erfolgt, die zueinander beabstandet sind, so dass jede Dosiervorrichtung oberhalb einer der Rillen (29) der unteren Bahn (20) angeordnet ist.

5. Anlage nach Anspruch 3 oder 4, wobei zur Kühlung der Schweißbereiche Kühlmittel mit Luftblasdüsen (50) vorgesehen sind, wobei die Kühlmittel mit Ablenkmitteln zum Schutz der pulvergefüllten Rillen (6) vor den Blasluftstrahlen versehen sind.

6. Anlage nach einem der Ansprüche 3 bis 5, ferner umfassend eine stromabwärts der Einheit zum Herstellen von Trockenkompressen (8) angeordnete Vakuum-Verpackungseinheit (14), wobei die Vakuum-Verpackungseinheit Mittel umfasst, um aufeinanderfolgend:
- einzelne Schalen (70) durch thermische Verformung herzustellen, zur Aufnahme der Trockenkompressen in einer ersten Bahn aus wasser- und luftdichtem Material (66), die zum Auslass der Einheit hin flachgezogen wird;
- in jeder Schale einen Wasserbeutel (10) und eine Trockenkompresse (1) übereinander zu legen;
- die Anordnung mit einer zweiten Bahn aus wasser- und luftdichtem Material (74) abzudecken, die zusammen mit der ersten Bahn zum Auslass der Einheit hin flachgezogen wird;
- die Ränder um jede Schale durch Zusammenschweißen der Bahnen mit Ausnahme des hinteren Rands der Schale zu verschließen;
- nacheinander in Laufrichtung der Anordnung stromaufwärts der Querverschweißung des hinteren Rands jeder Schale zu vakuumieren, bevor jede Schale durch Verschweißen des hinteren Rands vollständig verschlossen wird;
- die Anordnung mittels eines Ausstechers zu zerschneiden, um die vakuumverpackten Kühlkompressen als Einheiten zu erhalten.

7. Anlage nach Anspruch 6, wobei die thermische Verformung der Schalen (70) so dimensioniert ist, dass eine Trockenkompresse (1) und ein Wasserbeutel (10) vollständig im Volumen der Schale enthalten sind.

8. Anlage nach Anspruch 6 oder 7, umfassend Mittel zum Halten der Position des Wasserbeutels (10), um eine zentrale Position des Beutels in Bezug auf die Trockenkompresse (1) in der Verpackungshülle sicherzustellen.

9. Anlage nach einem der Ansprüche 6 bis 8, ferner umfassend eine Wasserbeutel-Herstellungseinheit, die stromaufwärts einer Verpackungseinheit (14) angeordnet ist, wobei die Wasserbeutel-Herstellungseinheit Mittel umfasst zum:
- Zusammenführen und Verschweißen der beiden seitlichen Ränder einer Kunststoffbahn zum Bilden eines in sich durch Verschweißen entlang eines Generators geschlossenen Schlauchs, wobei das Verschweißen ausgeführt wird, um einerseits den dichten Verschluss des Wasserbeutels (10) zu gewährleisten damit der Beutel späteren Manipulationen widersteht, und um andererseits einen Sollbruchbereich (12) zu bilden, der dazu ausgebildet ist, im fertigen Artikel nachzugeben, sobald flach auf den Artikel und damit auf den Wasserbeutel gedrückt wird, so dass das Wasser die Kompresse durchtränkt;
- Platzieren des so geformten Schlauchs in thermische Schweißbacken quer zur Achse des Schlauchs und Ausführen einer Querverschweißung dieses Schlauchs, die die untere Schweißlinie des Beutels bildet;
- Abgeben von Wasser unter Druck in aufeinanderfolgenden Dosen in der Mitte des Schlauchs, so dass sich der Schlauch mit Wasser füllt;
- Kontrollieren des Wasserfüllstands im Schlauch mit einer wasserstandsempfindlichen Sonde;
- wenn der Wasserstand einen vorbestimmten Schwellenwert erreicht hat, Ausführen der oberen Querverschweißung, indem dann ein Beutel gebildet wird, der das Wasser einschließt, wobei die obere Schweißlinie ausreichend unterhalb des oberen Wasserspiegels erfolgt, um überschüssiges Wasser auszutreiben und sicherzustellen, dass keine Luftblasen im Beutel eingeschlossen sind;
- Schneiden der so nacheinander gebildeten Wasserbeutel.

10. Anlage nach Anspruch 9, welche ferner Transfer-Mittel (62) für den Transfer der am Auslass der Einheit zum Herstellen von Trockenkompressen (8) erhaltenen Trockenkompressen (1) zum Einlass der Verpackungseinheit (14) umfasst, wobei die Transfer-Mittel dazu ausgebildet sind, ferner jeden Wasserbeutel (10) am Auslass der Einheit zum Herstellen von Trockenkompressen zu ergreifen, um eine Kompresse und einen zugehörigen Wasserbeutel übereinander in jede vorgeformte Schale (70) in der Verpackungshülle zu übertragen.

11. Anlage nach Anspruch 10, welche Mittel zur visiometrischen Kontrolle umfasst, um sicherzustellen, dass eine Dosis von absorbierendem Polymerpulver (6) im Inneren jeder Trockenkompresse (1) auf der Höhe der genannten Transfer-Mittel (62) vorhanden ist.

12. Kühlkompressen-Artikel (2), welcher durch Ausführen des Herstellungsverfahrens nach Anspruch 2 erhalten wird, umfassend, in steriler Verpackung in einer Vakuumhülle (66, 74), eine Kompresse (1) mit einem Kühleffekt durch Desorption von Wasser, welche Partikel eines absorbierenden Polymers im trockenen Zustand und einen Vorrat an aktivierendem Wasser (10) enthält, der in einem versiegelten Beutel zugeordnet ist, **dadurch gekennzeichnet, dass** die Trockenkompresse flach zwischen zwei Bahnen (20, 22) aus textilem Material zum Zurückhalten der absorbierenden Polymerpartikel angeordnet ist, die durch Laminierung zusammengefügt sind, von denen eine derart gewählt ist, dass sie wasserdurchlässig und thermisch auf reversible Weise verformbar ist, und dazu neigt, in ihre ursprüngliche Form zurückzukehren, und von denen die andere derart gewählt ist, dass sie gute mechanische Eigenschaften aufweist, insbesondere in Bezug auf die Beständigkeit gegen Zugbeanspruchung.

13. Artikel nach Anspruch 12, wobei die Vakuumverpackungshülle aus zwei Folien (66, 74) aus wasser- und luftdichtem Material gebildet ist, die an den Rändern jedes Artikels zusammenlaminiert und versiegelt sind, und wobei eine der Folien, die vorteilhafterweise an der Trockenkompresse platziert ist, aus einem Material besteht, das derart gewählt ist, dass es die Vorformung der Schalen durch permanente Heißverformung erleichtert, während die andere der Folien, die vorteilhafterweise an dem Wasserbeutel (10) platziert ist, aus einem Material besteht, das derart gewählt ist, dass es gute mechanische Eigenschaften aufweist, die einerseits die Festigkeit der Anordnung bei der Herstellung durch Laminierung gewährleisten und andererseits das Aufbrechen des Wasserbeutels durch manuellen Druck erleichtern.

## Claims

1. Process for manufacturing compresses having a cooling effect and containing absorbent polymer particles, consisting in laminating between two retention sheets (20, 22) of said particles which are continuously delivered along a dry compress manufacturing unit (8) in which successively: grooves (29) are formed in the sheet which is presented in (20) by inducing a reversible heat deformation by the application of heating pins (28) arranged in a line across the direction of travel, successive doses of absorbent polymer (36) in powder formed extending over a determined length of said grooves, the lower sheet so filled in is covered with the other said sheet coming as an upper sheet (22) ; the two sheets are welded (42) together along the intervals between adjacent grooves in order to close laterally the compartments filled with absorbent polymer; the end of said compartments is also welded (56) along weld lines transversal to the direction of travel, in the area in which the grooves are empty of the polymer between the successive doses deposited; the resulting assembly is then cut (60) into successive dry compresses along said weld lines.

2. Process according to claim 1, including a subsequent vacuum-packing step for said dry compresses according to which operation is made by lamination between of two watertight and airtight films, with a lower film in which receptacles are formed by hot plastic deformation and in which a compress and an associated water bag are deposited one on top of the other, with the water bag containing a predetermined quantity of water for the activation by swelling with water of all the absorbent polymer particles contained in the compress; the sheets are then welded together between the filled receptacles along longitudinal and transverse weld lines while forming a vacuum between two successive transverse weld operations and cutting the assembly along said longitudinal and transverse weld lines to separate the articles from each other.

3. A plant for implementing the process for manufacturing according to claim 1 or 2, comprising a large-scale manufacturing unit (8) wherein are provided:
- means to pass (21) continuously a water-permeable textile lower sheet (20) and delivered flat to the delivery end of the unit, underneath an absorbent particle (38) powder feed hopper (38);
- preforming pins (28), aligned transversally to the longitudinal direction of travel of the lower sheet, for deforming the said lower sheet into grooves (29) longitudinally formed said pins heating the taut lower sheet and deforming it mechanically by reversible thermal deformation, said grooves being able to receive successive doses of said powder (6)
- means for covering (23, 40, 43) the assembly with an upper sheet (22) which is continuously unwound and means for delivering the assembly formed by to the delivery end of said unit;
- first means for welding (42) able to weld together the upper sheet and the lower sheet along transversal weld lines, so as to isolate transversely from each other the other adjacent grooves filled with powder;
- second means for welding (56) able to weld the said sheets together
each other along transversal welding lines (54) transversally to the direction of travel, between the deposit sites of successive doses, so as to close into each groove successive compartments (4) containing each a dose of said powder of absorbent particulate;- means for cutting (60) of the assembly into successive dry compresses (1) by passing between roll one of which having a sharped blade (60).

4. Plant according to the previous claim in which the powder (6) is deposited by a set of fillers (36) spaced so that each filler is located above the grooves (29) in the moving lower sheet (20).

5. Plant according to claim 3 or 4, in which cooling means comprising blow tubes (50) are used to cool the welding areas, said cooling means being equipped with baffles to protect said powder-filled grooves (6) from blasts of air.

6. Plant according to any one of claims 3 to 5, further comprising a vacuum-packing unit (14) located downstream of the dry compress manufacturing unit (8), the said vacuum-packing unit comprising means for successively:
- to realize by thermal forming individual receptacles (70) to take dry compresses in a watertight, airtight sheet (66) which is delivered flat to the delivery end of the unit;
- to deposit a water bag (10) and dry compress (1) in each receptacle, one on top of the other;
- to cover the assembly with a second watertight, airtight sheet (74) which is delivered flat at the same time as the first sheet to the delivery end of the unit;
- to close the edges around each receptacle by welding the sheets together, except for the rear edge of said receptacle;
- to successively create a vacuum in the direction of travel of the assembly before transverse welding of the rear edge of each receptacle before the complete closure of each receptacle by welding of said rear edge;
- to cut the assembly with a punch to obtain individual vacuum packed compresses having a cooling effect.

7. Plant according to claim 6, in which construction of the receptacles (70) by thermic forming is designed such that a dry compress (1) and a water bag (10) are completely contained in the volume of said receptacle.

8. Plant according to claim 6 or 7, comprising means to hold the water bag (10) in a central position with respect to the dry compress (1) in the outer packaging.

9. Plant according to one of claims 6 to 8, further comprising a water bag manufacturing unit placed in front of a packaging unit (14), wherein means are provided to:
- bring together and weld the two sides of a plastic sheet to form a closed tube welded along a generatrix, said weld being carried out to ensure watertight closure of the water bag (10) and resistance to subsequent handling thereof and to form a frangible area (12) designed to give way, in the finished article, when the article and therefore the water bag, is pressed flat, so that the water impregnates the compress;
- place the resulting tube in thermic welding jaws perpendicular to the axis of the tube and transversely weld the tube to form the lower weld line of the bag;
- make flow the pressurized water in successive doses into the centre of the tube to fill it with water;
- check the filling level of the water in the tube by means of a water level probe;
- weld the water bag transversely when the water level has reached a predetermined level, thus forming a bag to enclose the water, with the upper weld line placed sufficiently below the upper level of the water to expel any excess water and ensure that no air bubbles are trapped in the bag;
- cut the water bags thus formed successively.

10. Plant according to claim 9, further including a means of transfer (62) to transfer the dry compresses (1) obtained at the delivery end of the dry compress manufacturing unit (8) to the feeding side of the packaging unit (14), with said means of transfer being designed to also pick up each water bag (10) at the delivery end of the water bag manufacturing unit, in order to transfer a compress and a water bag combined together in a pre-formed receptacle (70) in the outer packaging.

11. Plant according to claim 10 comprising videometric inspection equipment to ensure that a dose of absorbent polymer powder (6) is present inside each dry compress (1) at the site of said means of transfer (62).

12. Article of compresses having a cooling effect, obtained by the implementation of the process according to claim 2, comprising in sterile vacuum packaging (66; 74), a compress (1) having a cooling effect by desorption of water containing absorbent polymer particles in the dry state and activation water in a separate watertight bag (10), **characterized by** the fact that said dry compress is presented flat between two textile sheets (20, 22) of absorbent polymer particle retention material, assembled by lamination, one of which is chosen to be water permeable and reversibly heat deformable which has a tendency to recover its original shape and the other of which is chosen to have good mechanical properties, particularly tensile strength.

13. Article according to claim 12 in which said vacuum packaging consists of two watertight, airtight films (66;74) which are assembled by lamination and welded hermetically to each other along the edges of each article and in which one of said films, preferably placed in immediate proximity to the dry compress, is made in a material that facilitate the preforming of the receptacles by permanent heat deformation, when the other of said films, preferably placed in immediate proximity to the water bag (10) is made of a material chosen to have good mechanical properties ensuring resistance of the assembly during lamination and facilitating bursting of the water bag by manual pressure through it.
